(19) Europäisches Patentamt
European Patent Office
Office européen des brevets

(11) Veröffentlichungsnummer: **0 132 689**
**B1**

(12) # EUROPÄISCHE PATENTSCHRIFT

(45) Veröffentlichungstag der Patentschrift: **29.10.86**

(51) Int. Cl.[4]: **C 07 C 143/665**, C 07 C 139/00

(21) Anmeldenummer: **84108047.6**

(22) Anmeldetag: **10.07.84**

(54) **Verfahren zur Herstellung von 1-Amino-4-bromanthrachinon-2-sulfonsäure.**

(30) Priorität: **22.07.83 DE 3326563**

(43) Veröffentlichungstag der Anmeldung: **13.02.85 Patentblatt 85/7**

(45) Bekanntmachung des Hinweises auf die Patenterteilung: **29.10.86 Patentblatt 86/44**

(84) Benannte Vertragsstaaten: **CH DE FR GB LI**

(56) Entgegenhaltungen:
**CH - A - 549 625**
**DE - A - 2 551 767**
**DE - A - 2 740 885**
**DE - A - 2 740 889**
**DE - B - 2 303 746**
**US - A - 2 413 790**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Schmitz, Reinold, Dr., Im Kerberich 36, D-5068 Odenthal (DE)**
Erfinder: **Ohm, Josef, Dr., Tannenweg 16, D-5090 Leverkusen 3 (DE)**
Erfinder: **Schneider, Jürgen, Dr., Am Thelensiefen 9, D-5068 Odenthal (DE)**

**EP 0 132 689 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung ist ein verbessertes Verfahren zur Herstellung von 1-Amino-4-bromanthrachinon-2-sulfonsäure («Bromaminsäure») durch Einwirkung von elementarem Brom auf die wässrige Lösung von 1-Aminoanthrachinon-2-sulfonsäure («Aminsäure») bei üblichen Temperaturen und pH-Werten.

Bei der direkten Bromierung von Aminsäurelösungen entstehen bekanntlich grössere Mengen an unerwünschten Nebenprodukten (hauptsächlich 1-Amino-2,4-dibromanthrachinon), so dass bereits zahlreiche Verfahrensvarianten vorgeschlagen wurden, um die Ausbeute-mindernden Nebenreaktionen zu unterdrücken.

So wird beispielsweise gemäss FIAT 1313, II, Seite 214 empfohlen, die Aminsäure in Form einer feinen Suspension zu bromieren.

Gemäss DE-B 2 303 746, US-A 3 428 659 und GB-A 1 291 225 sollen die Nebenreaktionen dadurch vermieden werden können, dass man die Bromierung bei einem ganz bestimmten pH-Wert und/oder bei Gegenwart eines Oxidationsmittels (z.B. Chlor oder Hypochlorite) durchführt.

Weiterhin sind bereits Vorschläge gemacht worden, die Ausbeuten an Bromaminsäure durch Zusatz von organischen Basen (vgl. CS-A 122 371) oder Salzen organischer Säuren (vgl. JA 55/019 231) zu erhöhen.

Aus DE-A 2 551 767 ist schliesslich bekannt, das Brom gewissermassen in statu nascendi durch Verwendung einer Natriumbromid/-bromat-Mischung auf die Aminsäure einwirken zu lassen, um auf diese Weise zu hohen Ausbeuten zu gelangen.

Dennoch konnten alle diese Verfahren nicht voll befriedigen, da sie entweder eine sehr aufwendige Regeltechnik erfordern oder eine zu hohe Abwasserbelastung bewirken. Zum Teil wurden aber auch in den grosstechnischen Praxisversuchen einfach nicht die behaupteten hohen Ausbeuten erzielt.

Es wurde nun gefunden, dass man auch im grosstechnischen Massstab Bromaminsäure in hoher Ausbeute und Reinheit erhält, wenn man die Bromierung mit elementarem Brom in Gegenwart von Bromsäure oder deren Salzen durchführt.

Diese Umsetzung kann durch folgende Reaktionsgleichung näher veranschaulicht werden:

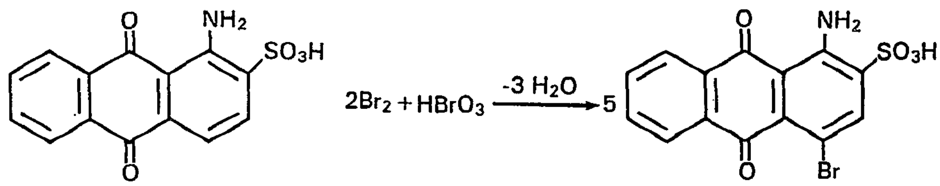


Besonders bemerkenswert ist dabei, dass pro Mol Aminsäure nur 1/5 Mol an der relativ teueren Bromsäuren benötigt wird, während das aus DE-A 2 551 767 bekannte «Bromsäure-Verfahren» 1/3 Mol dieser Säure erfordert.

Das Verfahren wird im übrigen — wie bereits erwähnt — unter Reaktionsbedingungen durchgeführt, die für die Bromierung von Aminsäure üblich sind, d.h. bei Temperaturen von —5 bis 50°C, vorzugsweise 20 - 30°C, und pH-Werten $< 4$ (vorzugsweise 0,5 - 3,0).

Man kann also die Reaktion ganz normal bei Raumtemperatur — ohne Kühlung oder Erhitzen — und in schwach saurem Milieu, wie es sich beim Eintragen der Aminsäure in Wasser von selbst einstellt, durchführen.

Ein besonderer Vorteil des Verfahrens besteht darin, dass man gemäss einer bevorzugten Variante die gesamte Menge an Bromsäure bzw. Bromat gemeinsam mit der Aminsäure vorlegen kann, ohne für eine genaue pH-Regelung und/oder Zudosierung des Oxidationsmittels wie bei einigen vorbeschriebenen Verfahren Sorge tragen zu müssen. Es muss lediglich darauf geachtet werden, dass das zugefügte elementare Brom rasch im Reaktionsgemisch verteilt wird, was man durch schnellaufende Rührer oder — im technischen Massstab — durch die Zugabe des Broms in eine geeignete Umpumpung des Kesselinhalts erreicht.

Die Aminsäure wird dabei zweckmässigerweise in Form des Natriumsalzes oder — vorzugsweise — als freie Säure, wie sie beispielsweise durch Sulfieren von 1-Aminoanthrachinon mit Chlorsulfonsäure in organischen Lösungsmitteln anfällt, eingesetzt. Beim Lösen der Rohsäure (Reinheitsgehalt ca. 90%) in Wasser stellt sich automatisch der gewünschte pH-Wert von 1 - 2 ein. Bevorzugt wird eine etwa 5- bis 10%ige Aminsäurelösung eingesetzt.

Dazu wird dann die berechnete Menge Bromsäure — vorzugsweise in Form des Na-Salzes — zugegeben. Ein geringer Überschuss oder Unterschuss an diesem Oxidationsmittel schadet jedoch nicht. Nach Zugabe der Bromsäure sollte man sofort mit der Bromierung beginnen.

Es empfiehlt sich, das Brom in genau der berechneten Menge zuzusetzen, da bei einem Überangebot an Brom die Bromaminsäure in 1-Amino-2,4-dibromanthrachinon umgewandelt wird. Der Fortgang der Bromierungsreaktion wird am besten dünnschichtchromatographisch verfolgt.

Die Aufarbeitung des Reaktionsgemischs kann auf verschiedene Weise erfolgen.

Insbesondere bei Verwendung von roher Aminsäure ist zunächst eine Klärung der Bromierungslösung mit üblichen Klärhilfsmitteln erforderlich. Zur Vermeidung von Korrosionsproblemen empfiehlt es sich jedoch, die saure Lösung zu neutralisieren und nach Zusatz der Hilfsmittel auf 80 - 100°C zu erhitzen.

Das Klärfiltrat kann gegebenenfalls mit wenig Natriumsulfat versetzt werden, um das Natriumsalz der Bromaminsäure besser zur Abscheidung zu bringen.

Die so gewonnene Bromaminsäure fällt in hervorragenden Ausbeuten (90 - 95% bezogen auf Aminsäure) und in hoher Reinheit an, so dass sie ohne weitere Reinigungsoperationen direkt in den diversen Farbstoffsynthesen eingesetzt werden kann.

Das nach Abtrennung der ausgeschiedenen Bromaminsäure erhaltene Filtrat ist ebenfalls sehr rein und kann daher direkt in Kläranlagen zur Aufbereitung abgeleitet werden.

*Beispiel*

57 g Aminsäure (90%ig) und 5,0 g Natriumbromat werden in 750 ml Wasser gelöst. Zu dieser Lösung werden bei Raumtemperatur unter kräftigem Rühren 4,1 ml Brom im Verlauf von 2 h zugetropft. Man lässt noch eine halbe Stunde nachrüihren. Im Dünnschichtchromatogramm sind dann weniger als 0,5% Aminsäure nachweisbar. Man setzt nun 4 g Aktivkohle und 2 g Kieselgur zu, neutralisiert mit verdünnter Natronlauge, heizt auf 95°C und filtriert das Unlösliche ab. Der Filterrückstand wird mit ca. 70 ml heissem Wasser gewaschen. Das mit der Mutterlauge vereinigte Waschfiltrat lässt man unter Erkalten über Nacht rühren.

Man isoliert durch Filtration 92% d.Th. reine Bromaminsäure. Im Klärrückstand befinden sich 1,2% d.Th. 1-Amino-2-bromanthrachinon und 1,8% d.Th. 1-Amino-2,4-dibromanthrachinon.

**Patentansprüche**

1. Verfahren zur Herstellung von 1-Amino-4-bromanthrachinon-2-sulfonsäure bzw. deren Salzen durch Einwirkung von elementarem Brom auf die wässrige Lösung von 1-Aminoanthrachinon-2-sulfonsäure bei üblichen Temperaturen und pH-Werten sowie in Gegenwart von Oxidationsmitteln, dadurch gekennzeichnet, dass man als Oxidationsmittel Bromsäure bzw. deren Salze verwendet.

2. Verfahren nach Anspruch 1, dadurch gekennzeichnet, dass man auf 1 Mol 1-Aminoanthrachinon-2-sulfonsäure 1/5 Mol Bromsäure einsetzt.

3. Verfahren nach Anspruch 1, dadurch gekennzeichnet, dass man die gesamte berechnete Bromsäuremenge und die 1-Aminoanthrachinon-2-sulfonsäure vorlegt und das Brom in unverdünnter Form unter guter Vermischung zusetzt.

**Claims**

1. Process for the preparation of 1-amino-4-bromoanthraquinone-2-sulphonic acid and its salts by reacting elemental bromine with an aqueous solution of 1-aminoanthraquinone-1-sulphonic acid at customary temperatures and pH values and in the presence of oxidising agents, characterised in that bromic acid or its salts are used as the oxidising agent.

2. Process according to claim 1, characterised in that 1/5 mole of bromic acid is used per 1 mole of 1-aminoanthraquinone-2-sulphonic acid.

3. Process according to claim 1, characterised in that the total calculated amount of bromic acid and the 1-aminoanthraquinone-2-sulphonic acid are initially introduced and the bromine is added in undiluted form with thorough mixing.

**Revendications**

1. Procédé de production d'acide 1-amino-4-bromanthraquinone-2-sulfonique et de ses sels par l'action du brome élémentaire sur une solution aqueuse d'acide 1-aminoanthraquinone-2-sulfonique aux températures et aux valeurs de pH classiques de même qu'en présence d'agents oxydants, caractérisé en ce qu'on utilise comme agents oxydants l'acide bromique ou ses sels.

2. Procédé suivant la revendciation 1, caractérisé en ce qu'on utilise 1/5 moles d'acide bromique par mole d'acide 1-aminoanthraquinone-2-sulfonique.

3. Procédé suivant la revendication 1, caractérisé en ce qu'on introduit préalablement la quantité calculée totale d'acide bromique et l'acide 1-aminoanthraquinone-2-sulfonique et on ajoute le brome sous la forme non diluée en mélangeant correctement.